# EUROPEAN PATENT SPECIFICATION

(11) **EP 3 528 782 B1**
(45) Date of publication and mention of the grant of the patent: **30.11.2022**
(21) Application number: 16795445.2
(22) Date of filing: 18.10.2016
(51) Int. Cl.: A61K 8/73, A61Q 17/00, A61K 8/02, A61K 8/20

(54) **METHOD OF PROTECTING KERATIN MATERIALS FROM POLLUTANTS**
VERFAHREN ZUM SCHÜTZEN VON KERATINMATERIALIEN VOR SCHADSTOFFEN
PROCÉDÉ DE PROTECTION DE MATIÈRES KÉRATINIQUES CONTRE LES POLLUANTS

(43) Date of publication of application: 28.08.2019
(73) Proprietor: L'OREAL, 75008 Paris (FR)
(72) Inventor: KOIKE, Toru, Kawasaki-shi Kanagawa 213-0012 (JP); HARADA, Yasuko, Kawasaki-shi Kanagawa 213-0012 (JP); YASUDA, Yoichi, Kawasaki-shi Kanagawa 213-0012 (JP)
(74) Representative: Nony
(86) International application number: PCT/JP2016/081378
(87) International publication number: WO 2018/073971

(56) References cited:
- EP-A1- 1 230 914
- WO-A1-2013/190709
- WO-A1-2016/098910
- US-A1- 2002 168 328
- US-A1- 2010 266 648
- Daito: "Cellulobeads", , 22 October 2008 (2008-10-22), XP002766861, Retrieved from the Internet: URL:http://daitokasei.com/img/e%20chemical /CELLULOBEADS%20BRO.pdf [retrieved on 2017-02-08]
- DATABASE GNPD [Online] MINTEL; June 2011 (2011-06), MST Beauty: "Treat & Conceal", XP002766862, Database accession no. 1579099
- KRUTMANN JEAN ET AL: "Pollution and skin: From epidemiological and mechanistic studies to clinical implications", JOURNAL OF DERMATOLOGICAL SCIENCE, ELSEVIER, AMSTERDAM, NL, vol. 76, no. 3, 16 September 2014 (2014-09-16), pages 163-168, XP029107620, ISSN: 0923-1811, DOI: 10.1016/J.JDERMSCI.2014.08.008
- ANDREA VIERKÖTTER ET AL: "Airborne Particle Exposure and Extrinsic Skin Aging", JOURNAL OF INVESTIGATIVE DERMATOLOGY, vol. 130, no. 12, 1 December 2010 (2010-12-01), pages 2719-2726, XP055341629, US ISSN: 0022-202X, DOI: 10.1038/jid.2010.204

## Description

### TECHNICAL FIELD

The present invention relates to a non-therapeutic method, preferably a cosmetic method, of protecting keratin materials from pollutants, the keratin materials being hair. The present invention further relates to the particles or the composition, preferably a cosmetic composition, comprising the particles as claimed for use in protecting keratin materials selected from the group consisting of skin, scalp, lips and hair.

### BACKGROUND ART

Urban environments are regularly subjected to peaks of pollution. An individual in his daily environment, and particularly in an urban zone, may be subjected to a whole range of factors attacking keratin materials, and in particular the skin, the scalp and the hair, by various airborne pollutants. Atmospheric pollutants which are represented largely by the primary and secondary products of combustion represent a major source of environmental oxidative stress. Urban pollution is composed of various types of chemical and xenobiotic products and particles. The major categories of pollutants which may exert harmful effects on the skin and the hair are as follows: gases, heavy metals, polycyclic aromatic hydrocarbons (PAHs) and particulate elements which are combustion residues onto which are adsorbed a very large number of organic and mineral compounds.

It is the outermost tissues that are initially and directly exposed to environmental toxins.

The skin is directly and frequently exposed to a pro-oxidative environment and it is particularly sensitive to the action of oxidative stress; its outermost layer serves as a barrier to oxidative damage which may take place. In the majority of circumstances, the oxidizing agent is generally neutralized after reaction with the keratin materials, but the reaction products formed may be responsible for attacks on cells and tissues. The stratum corneum, the skin's barrier, is the site of contact between the air and skin tissue, and the lipid/protein two-phase structure is a crucial factor of this barrier function of the skin. These elements may react with the oxidizing agents and become impaired, which will promote the desquamation phenomena.

Among the pollutants that may exert deleterious effects on keratin materials, toxic gases such as ozone, carbon monoxide, nitrogen oxides or sulphur oxides are among the major constituents of pollutants. It has been found that these toxic gases promote the desquamation of keratin materials; they "fatigue" the keratin materials, that is to say make them dull and dirty. Similarly, cellular asphyxia of the keratin materials has been observed.

It is known that heavy metals (lead, cadmium and mercury) are atmospheric pollutants whose emissions have increased considerably, especially in urban and industrial environments. Although the majority of the effects of these metals are seen in other tissues (lungs, kidneys, brain, etc.), it has been shown that certain metals can penetrate into the skin and become accumulated therein (A. B. G. Landsdown, Critical Reviews in Toxicology, 1995, Vol. 25, pp. 397462).

In addition to certain toxic effects which they cause, heavy metals have the property of reducing the activity of the cellular defense means against free radicals [see for example R. S. Dwivedi, J. Toxicol. Cut. & Ocular Toxical. 6(3), 183-191 (1987)). Thus, heavy metals aggravate the toxic effects of gaseous pollutants by reducing the efficacy of the natural defense means, and bring about an acceleration of the phenomenon of cell ageing. This is particularly true for keratin materials and especially the skin, the scalp and the hair, which are in direct and permanent contact with the external environment.

Another major category of pollutants consists of combustion residues in the form of particles onto which are adsorbed a very large number of organic compounds, and in particular of polycyclic aromatic hydrocarbons (PAHs). These PAHs adsorbed at the surface of the particles and dust borne by the urban atmosphere can penetrate into skin tissue and become stored and/or biotransformed therein.

Thus, the harmful effects of pollution on keratin materials affect cell respiration and are reflected by accelerated ageing of the skin, with a dull complexion and the early formation of wrinkles or fine lines, and also by a reduction in the vigour of the hair, which thus acquires a dull appearance. In addition, due to pollution, the skin and hair become dirty more quickly.

Various anti-pollution agents have been described to combat these effects of pollutants. Thus, document EP-A-557 042 describes the use of metallothionines to protect tissues against heavy metals. Moreover, document EP-A-577 718 describes the use of sphingolipids to protect the skin and the hair against atmospheric pollution.

Document US 2010/266648 is directed towards imparting superhydrophobic properties to cosmetics. It discloses a water-in-oil emulsion including (i) a continuous oil-phase; (ii) a discontinuous (internal) aqueous phase; (iii) a specific emulsifier; (iv) one or more hydrophobic film formers, and (v) one or more hydrophobic particulate materials having a median particle size between about 5 nm and about 1 mm.

However, this document does not disclose compositions comprising specific particles as defined in the present application, in particular in terms of wet point.

### DISCLOSURE OF INVENTION

With pollution on the increase, there is a need for other agents for effectively combating the harmful effect of pollutants on keratin materials and to prevent the adhesion of these pollutants on keratin materials, and in particular to avoid the degradation of cell respiration, the desquamation and accelerated ageing of keratin materials and especially the skin, and also to combat the dull complexion and the early formation of wrinkles and fine lines on the skin, to prevent hair from having a dull appearance and from becoming dirty, and to avoid irritation of the skin and also skin allergy phenomena and skin inflammation.

Thus, an objective of the present invention is to provide a method or process of protecting keratin materials from pollutants, as well as an anti-pollution agent and a use for the method or process.

When the keratin material is hair, the above objective of the present invention can be achieved by a non-therapeutic method, in particular a cosmetic method, of protecting keratin materials from pollutants, comprising: applying the keratin materials with at least one composition, preferably at least one cosmetic composition, comprising at least one particle having
a wet point for oil being at least 100 ml/100 g, preferably at least 150 ml/100 g, and more preferably at least 200 ml/100 g, and
a wet point for water being at least 100 ml/100 g, preferably at least 200 ml/100 g, and more preferably at least 300 ml/100 g.

Thus, the present invention concerns, according to one of its aspects, a non-therapeutic method, preferably a cosmetic method, of protecting keratin materials from pollutants, comprising:
applying the keratin materials with at least one composition, preferably at least one cosmetic composition, comprising at least one particle having
a wet point for oil being at least 100 ml/100 g, preferably at least 150 ml/100 g, and more preferably at least 200 ml/100 g, and
a wet point for water being at least 100 ml/100 g, preferably at least 200 ml/100 g, and more preferably at least 300 ml/100 g,
wherein the keratin materials are hair.

The pollutants may be aerial pollutants.

The pollutants may be selected from the group consisting of carbon black, carbon oxides, nitrogen oxides, sulfur oxides, hydrocarbons, organic volatiles, heavy metals, PM2.5 and mixtures thereof.

It is preferable that the number-average primary particle size of the particle used in the present invention be 50 µm or less, preferably 30 µm or less, and more preferably 10 µm or less.

It is preferable that the ratio of the wet point for water/the wet point for oil of the particle used in the present invention be 5 or less, preferably 4 or less, and more preferably 2 or less.

It is preferable that the particle used in the present invention be porous.

It is preferable that the particle be selected from the group consisting of polysaccharides, boron compounds, metal compounds, polymers, perlites, and mixtures thereof.

It is preferable that the particle used in the present invention comprise at least one polysaccharide, preferably cellulose.

It is also preferable that the particle used in the present invention comprises boron nitride.

The amount of the particle in the composition used in the present invention may be from 0.01 to 20% by weight, preferably from 0.1 to 15% by weight, and more preferably from 1.0 to 10% by weight relative to the total weight of the composition.

The keratin materials may be selected from the group consisting of skin, scalp, lips and hair.

Among the keratin materials skin can be cited, in particular face.

The skin may be protected from damage caused by pollutants selected from the group consisting of oily skin, dehydration of skin, alteration of desquamation, squalene decrease, vitamin E decrease, pigmentation, pore problems such as clogged pores, dilated pores, acne and black heads, loss of dry/oily balance, dull skin, aging, and lactic acid increase.

When the keratin materials are skin, scalp, lips and hair, the above objective of the present invention can also be achieved by a particle or a composition, preferably a cosmetic composition, comprising the particle, for use in protecting keratin materials from pollutants, wherein
the particle has
a wet point for oil being at least 100 ml/100 g, preferably at least 150 ml/100 g, and more preferably at least 200 ml/100 g, and
a wet point for water being at least 100 ml/100 g, preferably at least 200 ml/100 g, and more preferably at least 300 ml/100 g.

Thus, according to another aspect, the present invention relates to a particle or a composition, preferably a cosmetic composition, comprising the particle, for use in protecting keratin materials from pollutants, wherein
the particle has
a wet point for oil being at least 100 ml/100 g, preferably at least 150 ml/100 g, and more preferably at least 200 ml/100 g, and
a wet point for water being at least 100 ml/100 g, preferably at least 200 ml/100 g, and more preferably at least 300 ml/100 g.

According to this embodiment, the keratin materials may be selected from the group consisting of skin, scalp, lips and hair.

According to this embodiment, it is preferable that the keratin materials be skin, more preferably face, and that the skin be protected from damage caused by pollutants selected from the group consisting of oily skin, dehydration of skin, alteration of desquamation, squalene decrease, vitamin E decrease, pigmentation, pore problems such as clogged pores, dilated pores, acne and black heads, loss of dry/oily balance, dull skin, aging, and lactic acid increase.

The above objective of the present invention can also be achieved by a use of at least one particle or at least one composition comprising the at least one particle, for protecting keratin materials from pollutants, wherein the particle has
a wet point for oil being at least 100 ml/100 g, preferably at least 150 ml/100 g, and more preferably at least 200 ml/100 g, and
a wet point for water being at least 100 ml/100 g, preferably at least 200 ml/100 g, and more preferably at least 300 ml/100 g.

Thus, according to a third aspect, the present invention relates to a use of at least one particle or at least one composition comprising the at least one particle, for protecting keratin materials from pollutants, wherein the particle has
a wet point for oil being at least 100 ml/100 g, preferably at least 150 ml/100 g, and more preferably at least 200 ml/100 g, and
a wet point for water being at least 100 ml/100 g, preferably at least 200 ml/100 g, and more preferably at least 300 ml/100 g,
wherein the keratin materials are hair.

### BEST MODE FOR CARRYING OUT THE INVENTION

After diligent research, the inventors have now found, entirely surprisingly, that the use of at least one particle with specific wet points for oil and water, as well as the use of at least one composition including the particle(s), can address the above issues and in addition makes it possible to protect keratin materials against the effects of pollutants and especially of particulate pollutants.

The above particle or composition can be used for a method or process of protecting keratin materials from pollutants, as an anti-pollution agent. The expression "anti-pollution agent" means an agent which protects keratin materials so as to prevent, attenuate and/or eliminate the deleterious effects of pollutants (e.g. PAHs, heavy metals, etc.), especially those adsorbed onto particles.

Thus, the present invention can provide a method or process of protecting keratin materials from pollutants, as well as an anti-pollution agent and a use for the method or process.

Hereafter, each of the aspects of the present invention will be described in a detailed manner.

### [Method]

One of the aspects of the present invention is a non-therapeutic method, preferably a cosmetic method, of protecting keratin materials from pollutants, comprising:
applying the keratin materials with at least one composition, preferably at least one cosmetic composition, comprising at least one particle having
a wet point for oil being at least 100 ml/100 g, preferably at least 150 ml/100 g, and more preferably at least 200 ml/100 g, and
a wet point for water being at least 100 ml/100 g, preferably at least 200 ml/100 g, and more preferably at least 300 ml/100 g,
wherein the keratin materials are hair.

The type of the pollutants is not limited. However, it may be preferable that the pollutants be in the form of particles, and more preferably aerial pollutants which mean particulate pollutants which are present and drift in the air.

The pollutants may be selected from the group consisting of carbon black, carbon oxides (e.g., CO and CO2), nitrogen oxides (e.g., NO, NO2 and NOₓ), sulfur oxides (e.g., SO, SO2 and SOₓ), hydrocarbons (e.g., polycyclic aromatic hydrocarbons (PAHs)), organic volatiles, heavy metals, PM2.5, PM10 and mixtures thereof.

It is known that pollutants can cause oily skin, dehydration of skin and alteration of desquamation (cf. International Journal of Cosmetic Science, 2015, p. 1-10), squalene decrease (cf. International Journal of Cosmetic Science, 2015, p. 1-9), vitamin E decrease (FEBS let. 2000 Jan. 21; 466(1): 165-168), and pigmentation (J. Invest. Dermatol. 2010 Dec; 130(12):2719-2726).

The application of the composition can be performed by any means such as the hands. The amount of the composition to be applied onto the keratin substance is not limited. For example, however, 1 to 10 g of the composition may be applied.

### (Particle)

The particle used for the present invention has
a wet point for oil being at least 100 ml/100 g, preferably at least 150 ml/100 g, more preferably at least 200 ml/100 g, even more preferably at least 250 ml/100 g, and preferably 1500 ml/100g or less; and
a wet point for water being at least 100 ml/100 g, preferably at least 200 ml/100 g, more preferably at least 300 ml/100 g, even more preferably at least 350 ml/100 g, and preferably 1500 ml/100g or less.

The term "wet point for oil" in the specification means a quantity or amount of oil which is necessary to make a target powder completely wet, which can be recognized, in particular, by the formation of a paste with the target powder.

The wet point for oil can be determined by the following protocol.
(1) 2 g of a target powder is kneaded with a spatula on a glass plate while adding oil, in particular linear ester oil, such as isononyl isononanoate (WICKENOL 151 / ALZO).
(2) When the target powder becomes completely wet and starts to form a paste, the weight of the added oil is determined as the weight of wet point.
(3) The wet point for oil is calculated from the equation: Wet point for oil (ml/100 g) = {(the weight of wet point)/2 g}X100/the density of oil.

Similarly, the term "wet point for water" in the specification means a quantity or amount of water which is necessary to make a target powder completely wet, which can be recognized, in particular, by the formation of a paste with the target powder.

The wet point for water can be determined by the following protocol.
(1) 2 g of a target powder is kneaded with a spatula on a glass plate while adding water with a density of 0.998 g/ml.
(2) When the target powder becomes completely wet and starts to form a paste, the weight of the added water is determined as the weight of wet point.
(3) The wet point for water is calculated from the equation: Wet point for water (ml/100 g) = {(the weight of wet point)/2 g}X100/the density of water.

It is preferable that the ratio of the wet point for water/the wet point for oil of the particle used for the present invention be 5 or less, preferably 4 or less, more preferably 3 or less, and even more preferably 2 or less, and preferably 0.1 or more.

The particle size of the particle used for the present invention is not limited. However, it is preferable that the number-average primary particle size of the particle be 50 µm or less, preferably 30 µm or less, more preferably 10 µm or less, and even more preferably from 2 to 5 µm.

It is preferable, that 90 vol% or more of the particles used for the present invention have a number-average primary particle size ranging from 0.1 to 10 µm, preferably from 0.5 to 8 µm, and more preferably from 1 to 7 µm. If 90 vol% or more of the core particles have a number-average primary particle size ranging from 1 to 7 µm, optical effects due to the particles may also be achieved.

The number-average primary particle size can be measured by, for example, extracting and measuring from a photograph image obtained by SEM and the like, using a particle size analyzer such as a laser diffraction particle size analyzer, and the like. It is preferable to use a particle size analyzer such as a laser diffraction particle size analyzer.

It is preferable that the ratio of the longest diameter/the shortest diameter of the particle used for the present invention range from 1.0 to 10, preferably from 1.0 to 5, and more preferably from 1.0 to 3.

The particle used for the present invention may be porous or non-porous. It is preferable, however, that the particle used in the present invention be porous.

The porosity of the particle may be characterized by a specific surface area of from 0.05 m²/g to 1,500 m²/g, more preferably from 0.1 m²/g to 1,000 m²/g, and even more preferably from 0.2 m²/g to 500 m²/g according to the BET method.

The particle can comprise any materials, which are not limited to, and can be selected from polysaccharides such as cellulose; silicon compounds such as silica; boron compounds such as boron nitride; metal compounds such as alumina, barium sulfate and magnesium carbonate; polymers such as polyamide, especially Nylon, acrylic polymers, especially of polymethyl methacrylate, of polymethyl methacrylate/ethylene glycol dimethacrylate, of polyallyl methacrylate/ethylene glycol dimethacrylate or of ethylene glycol dimethacrylate/lauryl methacrylate copolymer; perlites; and mixtures thereof.

It is preferable that the particle be selected from the group consisting of polysaccharides, silicon compounds, boron compounds, metal compounds, polymers, perlites, and mixtures thereof.

The polysaccharide may be selected from alginic acid, guar gum, xanthan gum, gum arabic, arabinogalactan, carrageenan, agar, karaya gum, gum tragacanth, tara gum, pectin, locust bean gum, cardolan, gellan gum, dextran, pullulan, hyaluronic acid, cellulose and its derivatives, and mixtures thereof. Cellulose and its derivatives are preferable.

In the present invention, the cellulose that may be used is not limited by the types of cellulose such as cellulose I, cellulose II, or the like. As the cellulose which can be used as a material for the particle for the present invention, type II cellulose is preferable.

The cellulose which can be used as a material for the particle in the composition used for the present invention may be in any particulate form, in particular a spherical particle.

The cellulose particle, preferably spherical cellulose particle, can be prepared, for example, as follows.
(1) A slurry of calcium carbonate, as an aggregation inhibitor, is added to an alkaline water-soluble anionic polymer aqueous solution, and stirred.
(2) Viscose and the aqueous solution obtained in the above (1) are mixed to form a dispersion of viscose fine particles.
(3) The dispersion of viscose fine particles obtained in the above (2) is heated to aggregate the viscose in the dispersion, and neutralized with acid, to form cellulose fine particles.
(4) The cellulose fine particles are separated from the mother liquid obtained in the above (3), and washed and dried, if necessary.

The viscose is a raw material of the cellulose. It is preferable to use viscose with a gamma value of 30 to 100% by mass and an alkaline concentration of 4 to 10% by mass. As the above water-soluble anionic polymer, mention may be made of polyacrylic acid sodium salt, polystyrene sulfonic acid sodium salt, and the like. The above calcium carbonate is used to prevent the aggregation of viscose fine particles in the dispersion and to make the particle size of the cellulose particle smaller. As the calcium carbonate slurry, mention may be made of Tama Pearl TP-221GS marketed by Okutama Kogyo Co., Ltd. in Japan.

According to one embodiment, a cellulose derivative may be chosen from cellulose esters and ethers.

It is pointed out that the term "cellulose ester" means, in the text hereinabove and hereinbelow, a polymer consisting of an α (1-4) sequence of partially or totally esterified anhydroglucose rings, the esterification being obtained by reaction of all or only some of the free hydroxyl functions of the said anhydroglucose rings with a linear or branched carboxylic acid or carboxylic acid derivative (acid chloride or acid anhydride) containing from 1 to 4 carbon atoms.

Preferably, the cellulose ester results from the reaction of some of the free hydroxyl functions of the said rings with a carboxylic acid containing from 1 to 4 carbon atoms.

Advantageously, the cellulose esters are chosen from cellulose acetates, propionates, butyrates, isobutyrates, acetobutyrates and acetopropionates, and mixtures thereof.

These cellulose esters may have a weight-average molecular mass ranging from 3,000 to 1,000,000, preferably from 10,000 to 500,000 and more preferably from 15,000 to 300,000.

In the text hereinabove and hereinbelow, the term "cellulose ether" means a polymer consisting of an α (1-4) sequence of partially etherified anhydroglucose rings, some of the free hydroxyl functions of the said rings being substituted with a radical -OR, R preferably being a linear or branched alkyl radical containing from 1 to 4 carbon atoms.

The cellulose ethers are thus preferably chosen from cellulose alkyl ethers with an alkyl group containing from 1 to 4 carbon atoms, such as cellulose methyl, propyl, isopropyl, butyl and isobutyl ethers.

These cellulose ethers may have a weight-average molecular mass ranging from 3,000 to 1,000,000, preferably from 10,000 to 500,000 and more preferably from 15,000 to 300,000.

As the particle used for the present invention, mention may be made of, for example, the following spherical cellulose particles marketed by Daito Kasei in Japan:
Cellulobeads USF (wet point for oil is 296.0 ml/100 g, wet point for water is 400.8 ml/100 g, the ratio of the wet point for water/the wet point for oil is 1.4) with a particle size of 4 µm (porous cellulose).

It is also preferable that the particle used in the present invention comprise at least one silicon compound, preferably silicon oxide, and more preferably silica.

A silica suitable for the present invention is a hydrophilic silica selected from precipitated silicas, fumed silicas and mixtures thereof.

A silica suitable for the present invention may be spherical or non-spherical in shape, and may be porous or nonporous. In one of the embodiments of the present invention, a silica suitable for the present invention is spherical and porous. The porosity of a silica particle may be opened to the exterior or in the form of a central cavity.

A silica may be hydrophilic.

It is also preferable that the particle used in the present invention comprise boron nitride.

The most preferred form of boron nitride used for the powder in accordance with the present invention is hexagonal boron nitride. One suitable line of products is available as Combat^{®} boron nitride powders, from Standard Oil Engineered Materials Company, Niagara Falls, N.Y.; the high purity grades and specifically grade SHP3, are preferred.

The particle used for the present invention may or may not be coated beforehand.

In a particular embodiment, the particle is originally coated. The material of an original coating of the particle is not limited, but an organic material such as a mono- or di-carboxylic acid or a salt thereof, an amino acid, an N-acylamino acid, an amido, a silicone and a modified silicone, may be preferable. As the organic material, mention may be made of potassium succinate, lauroyl lysine and acryl-modified silicone.

In other words, the particle used for the present invention may be surface-treated. As examples of the surface treatments, mention may be made of the following:
(a) Fluorine-based compound treatments such as treatments with perfluoroalkylphosphates, perfluoroalkylsilanes, perfluoropolyethers, fluorosilicones, and fluorinated silicone resins
(b) Silicone treatments such as treatments with methylhydrogenpolysiloxanes, dimethylpolysiloxanes, and tetramethyltetrahydrogencyclotetrasiloxane in gas phase
(c) Pendant treatments such as treatments to add an alkyl chain and the like after the gas phase silicone treatment
(d) Silane coupling agent treatments
(e) Titanium coupling agent treatments
(f) Aluminum coupling agent treatments
(g) Oil agent treatments
(h) N-acylated lysine treatments
(i) Polyacrylic acid treatments
(j) Metal soap treatments such as those with stearate salt or myristate salt
(k) Acrylic resin treatments
(l) Metal oxide treatments

It is possible to perform a plurality of surface treatments in combination with the above treatments.

As the particle used for the present invention, Cellulobeads USF, Sunsphere H33 and Boron Nitride SHP3 are preferable. Cellulobeads USF and Sunsphere H33 are more preferable, and Cellulobeads USF is most preferable.

### (Composition)

The composition used for the present invention includes at least one particle as explained above. If two or more particles are used, they may be the same or different.

The amount of the particle in the composition used for the present invention may be from 0.01 to 20% by weight, preferably from 0.1 to 15% by weight, and more preferably from 1.0 to 10% by weight relative to the total weight of the composition.

The composition according to the present invention may comprise at least one oil. If two or more oils are used, they may be the same or different.

Here, "oil" means a fatty compound or substance which is in the form of a liquid or a paste (non-solid) at room temperature (25°C) under atmospheric pressure (760 mmHg). As the oils, those generally used in cosmetics can be used alone or in combination thereof. These oils may be volatile or non-volatile.

The oil may be a non-polar oil such as a hydrocarbon oil, a silicone oil, or the like; a polar oil such as a plant or animal oil and an ester oil or an ether oil; or a mixture thereof.

The oil may be selected from the group consisting of oils of plant or animal origin, synthetic oils, silicone oils, hydrocarbon oils and fatty alcohols.

It is preferable that the oil be selected from synthetic oils, hydrocarbon oils, and mixtures thereof, more preferably from ester oils, hydrocarbon oils and mixtures thereof, and even more preferably from ester oils.

As examples of plant oils, mention may be made of, for example, linseed oil, camellia oil, macadamia nut oil, corn oil, mink oil, olive oil, avocado oil, sasanqua oil, castor oil, safflower oil, jojoba oil, sunflower oil, almond oil, rapeseed oil, sesame oil, soybean oil, peanut oil, and mixtures thereof.

As examples of animal oils, mention may be made of, for example, squalene and squalane.

As examples of synthetic oils, mention may be made of alkane oils such as isododecane and isohexadecane, ester oils, ether oils, and artificial triglycerides.

The ester oils are preferably liquid esters of saturated or unsaturated, linear or branched C1-C26 aliphatic monoacids or polyacids and of saturated or unsaturated, linear or branched C1-C26 aliphatic monoalcohols or polyalcohols, the total number of carbon atoms of the esters being greater than or equal to 10.

Preferably, for the esters of monoalcohols, at least one from among the alcohol and the acid from which the esters of the present invention are derived is branched.

Among the monoesters of monoacids and of monoalcohols, mention may be made of ethyl palmitate, ethyl hexyl palmitate, isopropyl palmitate, dicaprylyl carbonate, alkyl myristates such as isopropyl myristate or ethyl myristate, isocetyl stearate, 2-ethylhexyl isononanoate, isononyl isononanoate, isodecyl neopentanoate and isostearyl neopentanoate.

Esters of C₄-C₂₂ dicarboxylic or tricarboxylic acids and of C₁-C₂₂ alcohols, and esters of monocarboxylic, dicarboxylic or tricarboxylic acids and of non-sugar C₄-C₂₆ dihydroxy, trihydroxy, tetrahydroxy or pentahydroxy alcohols may also be used.

Mention may especially be made of: diethyl sebacate; isopropyl lauroyl sarcosinate; diisopropyl sebacate; bis(2-ethylhexyl) sebacate; diisopropyl adipate; di-n-propyl adipate; dioctyl adipate; bis(2-ethylhexyl) adipate; diisostearyl adipate; bis(2-ethylhexyl) maleate; triisopropyl citrate; triisocetyl citrate; triisostearyl citrate; glyceryl trilactate; glyceryl trioctanoate; trioctyldodecyl citrate; trioleyl citrate; neopentyl glycol diheptanoate; diethylene glycol diisononanoate.

As ester oils, one can use sugar esters and diesters of C₆-C₃₀ and preferably C₁₂-C₂₂ fatty acids. It is recalled that the term "sugar" means oxygen-bearing hydrocarbon-based compounds containing several alcohol functions, with or without aldehyde or ketone functions, and which comprise at least 4 carbon atoms. These sugars may be monosaccharides, oligosaccharides or polysaccharides.

Examples of suitable sugars that may be mentioned include sucrose (or saccharose), glucose, galactose, ribose, fucose, maltose, fructose, mannose, arabinose, xylose and lactose, and derivatives thereof, especially alkyl derivatives, such as methyl derivatives, for instance methylglucose.

The sugar esters of fatty acids may be chosen especially from the group comprising the esters or mixtures of esters of sugars described previously and of linear or branched, saturated or unsaturated C₆-C₃₀ and preferably C₁₂-C₂₂ fatty acids. If they are unsaturated, these compounds may have one to three conjugated or non-conjugated carbon-carbon double bonds.

The esters according to this variant may also be selected from monoesters, diesters, triesters, tetraesters and polyesters, and mixtures thereof.

These esters may be, for example, oleates, laurates, palmitates, myristates, behenates, cocoates, stearates, linoleates, linolenates, caprates and arachidonates, or mixtures thereof such as, especially, oleopalmitate, oleostearate and palmitostearate mixed esters, as well as pentaerythrityl tetraethyl hexanoate.

More particularly, use is made of monoesters and diesters and especially sucrose, glucose or methylglucose monooleates or dioleates, stearates, behenates, oleopalmitates, linoleates, linolenates and oleostearates.

An example that may be mentioned is the product sold under the name Glucate^{®} DO by the company Amerchol, which is a methylglucose dioleate.

As examples of preferable ester oils, mention may be made of, for example, diisopropyl adipate, dioctyl adipate, 2-ethylhexyl hexanoate, ethyl laurate, cetyl octanoate, octyldodecyl octanoate, isodecyl neopentanoate, myristyl propionate, 2-ethylhexyl 2-ethylhexanoate, 2-ethylhexyl octanoate, 2-ethylhexyl caprylate/caprate, methyl palmitate, ethyl palmitate, isopropyl palmitate, dicaprylyl carbonate, isopropyl lauroyl sarcosinate, isononyl isononanoate, ethylhexyl palmitate, isohexyl laurate, hexyl laurate, isocetyl stearate, isopropyl isostearate, isopropyl myristate, isodecyl oleate, glyceryl tri(2-ethylhexanoate), pentaerythrithyl tetra(2-ethylhexanoate), 2-ethylhexyl succinate, diethyl sebacate, and mixtures thereof.

As examples of artificial triglycerides, mention may be made of, for example, capryl caprylyl glycerides, glyceryl trimyristate, glyceryl tripalmitate, glyceryl trilinolenate, glyceryl trilaurate, glyceryl tricaprate, glyceryl tricaprylate, glyceryl tri(caprate/caprylate) and glyceryl tri(caprate/caprylate/linolenate).

As examples of silicone oils, mention may be made of, for example, linear organopolysiloxanes such as dimethylpolysiloxane, methylphenylpolysiloxane, methylhydrogenpolysiloxane, and the like; cyclic organopolysiloxanes such as cyclohexasiloxane, octamethylcyclotetrasiloxane, decamethylcyclopentasiloxane, dodecamethylcyclohexasiloxane, and the like; and mixtures thereof.

Preferably, the silicone oil is chosen from liquid polydialkylsiloxanes, especially liquid polydimethylsiloxanes (PDMS) and liquid polyorganosiloxanes comprising at least one aryl group.

These silicone oils may also be organomodified. The organomodified silicones that can be used according to the present invention are silicone oils as defined above and comprise in their structure one or more organofunctional groups attached via a hydrocarbon-based group.

Organopolysiloxanes are defined in greater detail in Walter Noll's Chemistry and Technology of Silicones (1968), Academic Press. They may be volatile or non-volatile.

When they are volatile, the silicones are more particularly chosen from those having a boiling point of between 60°C and 260°C, and even more particularly from:
(i) cyclic polydialkylsiloxanes comprising from 3 to 7 and preferably 4 to 5 silicon atoms. These are, for example, octamethylcyclotetrasiloxane sold in particular under the name Volatile Silicone^{®} 7207 by Union Carbide or Silbione^{®} 70045 V2 by Rhodia, decamethylcyclopentasiloxane sold under the name Volatile Silicone^{®} 7158 by Union Carbide, Silbione^{®} 70045 V5 by Rhodia, and dodecamethylcyclopentasiloxane sold under the name Silsoft 1217 by Momentive Performance Materials, and mixtures thereof. Mention may also be made of cyclocopolymers of the type such as dimethylsiloxane/methylalkylsiloxane, such as Silicone Volatile^{®} FZ 3109 sold by the company Union Carbide, of formula: 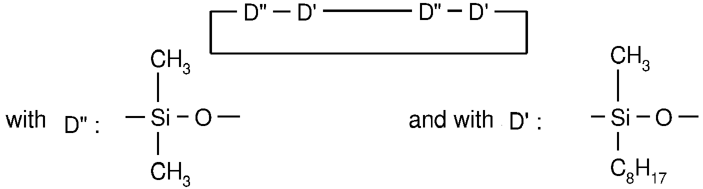 Mention may also be made of mixtures of cyclic polydialkylsiloxanes with organosilicon compounds, such as the mixture of octamethylcyclotetrasiloxane and tetratrimethylsilylpentaerythritol (50/50) and the mixture of octamethylcyclotetrasiloxane and oxy-1,1'-bis(2,2,2',2',3,3'-hexatrimethylsilyloxy)neopentane; and
(ii) linear volatile polydialkylsiloxanes containing 2 to 9 silicon atoms and having a viscosity of less than or equal to 5×10⁻⁶ m²/s at 25°C. An example is decamethyltetrasiloxane sold in particular under the name SH 200 by the company Toray Silicone. Silicones belonging to this category are also described in the article published in Cosmetics and Toiletries, Vol. 91, Jan. 76, pp. 27-32, Todd & Byers, Volatile Silicone Fluids for Cosmetics*.* The viscosity of the silicones is measured at 25°C according to ASTM standard 445 Appendix C.

Non-volatile polydialkylsiloxanes may also be used. These non-volatile silicones are more particularly chosen from polydialkylsiloxanes, among which mention may be made mainly of polydimethylsiloxanes containing trimethylsilyl end groups.

Among these polydialkylsiloxanes, mention may be made, in a non-limiting manner, of the following commercial products:
- the Silbione^{®} oils of the 47 and 70 047 series or the Mirasil^{®} oils sold by Rhodia, for instance the oil 70 047 V 500 000;
- the oils of the Mirasil^{®} series sold by the company Rhodia;
- the oils of the 200 series from the company Dow Corning, such as DC200 with a viscosity of 60 000 mm²/s; and
- the Viscasil^{®} oils from General Electric and certain oils of the SF series (SF 96, SF 18) from General Electric.

Mention may also be made of polydimethylsiloxanes containing dimethylsilanol end groups known under the name dimethiconol (CTFA), such as the oils of the 48 series from the company Rhodia.

Among the silicones containing aryl groups, mention may be made of polydiarylsiloxanes, especially polydiphenylsiloxanes and polyalkylarylsiloxanes such as phenyl silicone oil.

The phenyl silicone oil may be chosen from the phenyl silicones of the following formula: 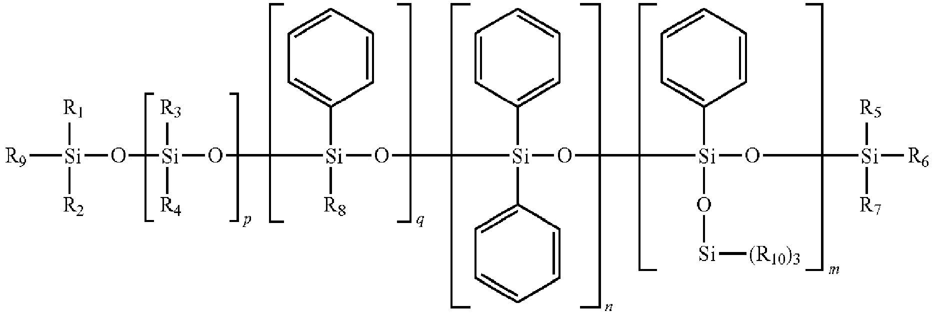 in which
R₁ to R₁₀, independently of each other, are saturated or unsaturated, linear, cyclic or branched C₁-C₃₀ hydrocarbon-based radicals, preferably C₁-C₁₂ hydrocarbon-based radicals, and more preferably C₁-C₆ hydrocarbon-based radicals, in particular methyl, ethyl, propyl or butyl radicals, and
m, n, p and q are, independently of each other, integers from 0 to 900 inclusive, preferably 0 to 500 inclusive, and more preferably 0 to 100 inclusive,
with the proviso that the sum n+m+q is other than 0.

Examples that may be mentioned include the products sold under the following names:
- the Silbione^{®} oils of the 70 641 series from Rhodia;
- the oils of the Rhodorsil^{®} 70 633 and 763 series from Rhodia;
- the oil Dow Corning 556 Cosmetic Grade Fluid from Dow Corning;
- the silicones of the PK series from Bayer, such as the product PK20;
- certain oils of the SF series from General Electric, such as SF 1023, SF 1154, SF 1250 and SF 1265.

As the phenyl silicone oil, phenyl trimethicone (R₁ to R₁₀ are methyl; p, q, and n = 0; m=1 in the above formula) is preferable.

The organomodified liquid silicones may especially contain polyethyleneoxy and/or polypropyleneoxy groups. Mention may thus be made of the silicone KF-6017 proposed by Shin-Etsu, and the oils Silwet^{®} L722 and L77 from the company Union Carbide.

Hydrocarbon oils may be chosen from:
- linear or branched, optionally cyclic, C₆-C₁₆ lower alkanes. Examples that may be mentioned include hexane, undecane, dodecane, tridecane, and isoparaffins, for instance isohexadecane, isododecane and isodecane; and
- linear or branched hydrocarbons containing more than 16 carbon atoms, such as liquid paraffins, liquid petroleum jelly, polydecenes and hydrogenated polyisobutenes such as Parleam^{®}, and squalane.

As preferable examples of hydrocarbon oils, mention may be made of, for example, linear or branched hydrocarbons such as isohexadecane, isododecane, squalane, mineral oil (e.g., liquid paraffin), paraffin, vaseline or petrolatum, naphthalenes, and the like; hydrogenated polyisobutene, isoeicosan, and decene/butene copolymer; and mixtures thereof.

The term "fatty" in the fatty alcohol means the inclusion of a relatively large number of carbon atoms. Thus, alcohols which have 4 or more, preferably 6 or more, and more preferably 12 or more carbon atoms are encompassed within the scope of fatty alcohols. The fatty alcohol may be saturated or unsaturated. The fatty alcohol may be linear or branched.

The fatty alcohol may have the structure R-OH wherein R is chosen from saturated and unsaturated, linear and branched radicals containing from 4 to 40 carbon atoms, preferably from 6 to 30 carbon atoms, and more preferably from 12 to 20 carbon atoms. In at least one embodiment, R may be chosen from C₁₂-C₂₀ alkyl and C₁₂-C₂₀ alkenyl groups. R may or may not be substituted with at least one hydroxyl group.

As examples of the fatty alcohol, mention may be made of lauryl alcohol, cetyl alcohol, stearyl alcohol, isostearyl alcohol, behenyl alcohol, undecylenyl alcohol, myristyl alcohol, octyldodecanol, hexyldecanol, oleyl alcohol, linoleyl alcohol, palmitoleyl alcohol, arachidonyl alcohol, erucyl alcohol, and mixtures thereof.

It is preferable that the fatty alcohol be a saturated fatty alcohol.

Thus, the fatty alcohol may be selected from straight or branched, saturated or unsaturated C₆-C₃₀ alcohols, preferably straight or branched, saturated C₆-C₃₀ alcohols, and more preferably straight or branched, saturated C₁₂-C₂₀ alcohols.

The term "saturated fatty alcohol" here means an alcohol having a long aliphatic saturated carbon chain. It is preferable that the saturated fatty alcohol be selected from any linear or branched, saturated C₆-C₃₀ fatty alcohols. Among the linear or branched, saturated C₆-C₃₀ fatty alcohols, linear or branched, saturated C₁₂-C₂₀ fatty alcohols may preferably be used. Any linear or branched, saturated C₁₆-C₂₀ fatty alcohols may be more preferably used. Branched C₁₆-C₂₀ fatty alcohols may be even more preferably used.

As examples of saturated fatty alcohols, mention may be made of lauryl alcohol, cetyl alcohol, stearyl alcohol, isostearyl alcohol, behenyl alcohol, undecylenyl alcohol, myristyl alcohol, octyldodecanol, hexyldecanol, and mixtures thereof. In one embodiment, cetyl alcohol, stearyl alcohol, octyldodecanol, hexyldecanol, or a mixture thereof (e.g., cetearyl alcohol) as well as behenyl alcohol, can be used as a saturated fatty alcohol.

According to at least one embodiment, the fatty alcohol used in the composition according to the present invention is preferably chosen from octyldodecanol, hexyldecanol and mixtures thereof.

The amount of the oil(s) in the composition used for the present invention may range from 1 to 90% by weight, preferably from 20 to 80% by weight, and more preferably from 30 to 70% by weight, relative to the total weight of the composition.

The composition according to the present invention may include at least one UV filter. If two or more UV filters are used, they may be the same or different.

The UV filter may be solid or liquid, preferably liquid. The terms "solid" and "liquid" mean solid and liquid, respectively, at 25°C under 1 atm. The UV filter may be made from at least one organic or inorganic material, preferably at least one organic material. Thus, the UV filter is preferably an organic UV filter.

The organic UV filter may be selected from the group consisting of anthranilic derivatives; dibenzoylmethane derivatives; cinnamic derivatives; salicylic derivatives; camphor derivatives; benzophenone derivatives; β,β-diphenylacrylate derivatives; triazine derivatives; benzotriazole derivatives; benzalmalonate derivatives; benzimidazole derivatives; imidazoline derivatives; bis-benzoazolyl derivatives; p-aminobenzoic acid (PABA) and derivatives thereof; benzoxazole derivatives; screening polymers and screening silicones; dimers derived from α-alkylstyrene; 4,4-diarylbutadienes; octocrylene and derivatives thereof, guaiazulene and derivatives thereof, rutin and derivatives thereof, flavonoids, biflavonoids, oryzanol and derivatives thereof, quinic acid and derivatives thereof, phenols, retinol, cysteine, aromatic amino acids, peptides having an aromatic amino acid residue, and mixtures thereof.

Mention may be made, as examples of the organic UV filter, of those denoted below under their INCI names, and mixtures thereof.
- Anthranilic derivatives: Menthyl anthranilate, marketed under the trademark "Neo Heliopan MA" by Haarmann and Reimer.
- Dibenzoylmethane derivatives: Butyl methoxydibenzoylmethane, marketed in particular under the trademark "Parsol 1789" by Hoffmann-La Roche; and isopropyl dibenzoylmethane.
- Cinnamic derivatives: Ethylhexyl methoxycinnamate, marketed in particular under the trademark "Parsol MCX" by Hoffmann-La Roche; isopropyl methoxycinnamate; isopropoxy methoxycinnamate; isoamyl methoxycinnamate, marketed under the trademark "Neo Heliopan E 1000" by Haarmann and Reimer; cinoxate (2-ethoxyethyl-4-methoxy cinnamate); DEA methoxycinnamate; diisopropyl methylcinnamate; and glyceryl ethylhexanoate dimethoxycinnamate.
- Salicylic derivatives: Homosalate (homomentyl salicylate), marketed under the trademark "Eusolex HMS" by Rona/EM Industries; ethylhexyl salicylate, marketed under the trademark "Neo Heliopan OS" by Haarmann and Reimer; glycol salicylate; butyloctyl salicylate; phenyl salicylate; dipropyleneglycol salicylate, marketed under the trademark "Dipsal" by Scher; and TEA salicylate, marketed under the trademark "Neo Heliopan TS" by Haarmann and Reimer.
- Camphor derivatives, in particular, benzylidenecamphor derivatives: 3-benzylidene camphor, manufactured under the trademark "Mexoryl SD" by Chimex; 4-methylbenzylidene camphor, marketed under the trademark "Eusolex 6300" by Merck; benzylidene camphor sulfonic acid, manufactured under the trademark "Mexoryl SL" by Chimex; camphor benzalkonium methosulfate, manufactured under the trademark "Mexoryl SO" by Chimex; terephthalylidene dicamphor sulfonic acid, manufactured under the trademark "Mexoryl SX" by Chimex; and polyacrylamidomethyl benzylidene camphor, manufactured under the trademark "Mexoryl SW" by Chimex.
- Benzophenone derivatives: Benzophenone-1 (2,4-dihydroxybenzophenone), marketed under the trademark "Uvinul 400" by BASF; benzophenone-2 (Tetrahydroxybenzophenone), marketed under the trademark "Uvinul D50" by BASF; Benzophenone-3 (2-hydroxy-4-methoxybenzophenone) or oxybenzone, marketed under the trademark "Uvinul M40" by BASF; benzophenone-4 (hydroxymethoxy benzophonene sulfonic acid), marketed under the trademark "Uvinul MS40" by BASF; benzophenone-5 (Sodium hydroxymethoxy benzophenone Sulfonate); benzophenone-6 (dihydroxy dimethoxy benzophenone); marketed under the trademark "Helisorb 11" by Norquay; benzophenone-8, marketed under the trademark "Spectra-Sorb UV-24" by American Cyanamid; benzophenone-9 (Disodium dihydroxy dimethoxy benzophenonedisulfonate), marketed under the trademark "Uvinul DS-49" by BASF; benzophenone-12, and n-hexyl 2-(4-diethylamino-2-hydroxybenzoyl)benzoate.
- β,β-Diphenylacrylate derivatives: Octocrylene, marketed in particular under the trademark "Uvinul N539" by BASF; and Etocrylene, marketed in particular under the trademark "Uvinul N35" by BASF.
- Triazine derivatives: diethylhexyl butamido triazone, marketed under the trademark "Uvasorb HEB" by Sigma 3V; 2,4,6-tris(dineopentyl 4'-aminobenzalmalonate)-s-triazine.
- Benzotriazole derivatives, in particular, phenylbenzotriazole derivatives: 2-(2H-benzotriazole-2-yl)-6-dodecyl-4-methylphenol, branched and linear; and those described in USP 5240975.
- Benzalmalonate derivatives: Dineopentyl 4'-methoxybenzalmalonate, and polyorganosiloxane comprising benzalmalonate functional groups, such as polysilicone-15, marketed under the trademark "Parsol SLX" by Hoffmann-LaRoche.
- Benzimidazole derivatives, in particular, phenylbenzimidazole derivatives: Phenylbenzimidazole sulfonic acid, marketed in particular under the trademark "Eusolex 232" by Merck, and disodium phenyl dibenzimidazole tetrasulfonate, marketed under the trademark "Neo Heliopan AP" by Haarmann and Reimer.
- Imidazoline derivatives: Ethylhexyl dimethoxybenzylidene dioxoimidazoline propionate.
- Bis-benzoazolyl derivatives: The derivatives as described in EP-669,323 and U.S. Pat. No. 2,463,264.
- Para-aminobenzoic acid and derivatives thereof: PABA (p-aminobenzoic acid), ethyl PABA, Ethyl dihydroxypropyl PABA, pentyl dimethyl PABA, ethylhexyl dimethyl PABA, marketed in particular under the trademark "Escalol 507" by ISP, glyceryl PABA, and PEG-25 PABA, marketed under the trademark "Uvinul P25" by BASF.
- Methylene bis-(hydroxyphenylbenzotriazol) compounds, such as 2,2'-methylenebis[6-(2H-benzotriazol-2-yl)-4-methyl-phenol] marketed in the solid form under the trademark "Mixxim BB/200" by Fairmount Chemical, 2,2'-methylenebis[6-(2H-benzotriazol-2-yl)-4-(1,1,3,3-tetramethylbutyl)phenol] marketed in the micronized form in aqueous dispersion under the trademark "Tinosorb M" by BASF, or under the trademark "Mixxim BB/100" by Fairmount Chemical, and the derivatives as described in U.S. Pat. Nos. 5,237,071 and 5,166,355, GB-2,303,549, DE-197,26,184 and EP-893,119, and Drometrizole trisiloxane, marketed under the trademark "Silatrizole" by Rhodia Chimie or "Mexoryl XL" by L'Oreal, as represented below. 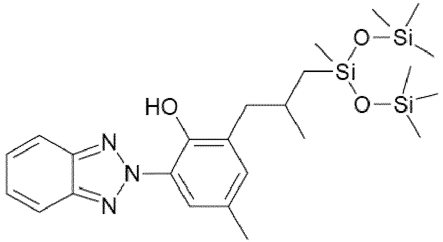
- Benzoxazole derivatives: 2,4-bis[5-1(dimethylpropyl)benzoxazol-2-yl-(4-phenyl)imino]-6-(2-ethylhexyl)imino-1,3,5-tri azine, marketed under the trademark of Uvasorb K2A by Sigma 3V.
- Screening polymers and screening silicones: The silicones described in WO 93/04665.
- Dimers derived from α-alkylstyrene: The dimers described in DE-19855649.
- 4,4-Diarylbutadiene derivatives: 1,1-dicarboxy(2,2'-dimethylpropyl)-4,4-diphenylbutadiene.
- Guaiazulene and derivatives thereof: Guaiazulene and sodium guaiazulene sulfonate.
- Rutin and derivatives thereof: Rutin and glucosylrutin.
- Flavonoids: Robustin (isoflavonoid), genistein (flavonoid), tectochrysin (flavonoid), and hispidone (flavonoid).
- Biflavonoids: Lanceolatin A, lanceolatin B, and hypnumbiflavonoid A.
- Oryzanol and derivatives thereof: Γ-oryzanol.
- Quinic acid and derivatives thereof: Quinic acid.
- Phenols: Phenol.
- Retinols: Retinol.
- Cysteines: L-cysteine.
- Peptides having an aromatic amino acid residue: Peptides having tryptophan, tyrosine or phenylalanine.

The preferred organic UV filter may be selected from:
butyl methoxydibenzoylmethane, ethylhexyl methoxycinnamate, homosalate, ethylhexyl salicylate, octocrylene, phenylbenzimidazole sulfonic acid, benzophenone-3, benzophenone-4, benzophenone-5, n-hexyl 2-(4-diethylamino-2-hydroxybenzoyl)benzoate, 4-methylbenzylidene camphor, terephthalylidene dicamphor sulfonic acid, disodium phenyl dibenzimidazole tetrasulfonate, ethylhexyl triazone, bis-ethylhexyloxyphenol methoxyphenyl triazine, diethylhexyl butamido triazone, 2,4,6-tris(dineopentyl 4'-aminobenzalmalonate)-s-triazine, 2,4,6-tris(diisobutyl 4'-aminobenzalmalonate)-s-triazine, methylene bis-benzotriazolyl tetramethylbutylphenol, polysilicone-15, dineopentyl 4'-methoxybenzalmalonate, 1,1-dicarboxy(2,2'-dimethylpropyl)-4,4-diphenylbutadiene, 2,4-bis[5-1(dimethylpropyl)benzoxazol-2-yl-(4-phenyl)imino]-6-(2-ethylhexyl)imino-1,3,5-tri azine, and their mixtures. A more preferable organic UV filter is butyl methoxydibenzoylmethane (Avobenzone).

In a preferred embodiment, the (d) UV filter is an organic liquid UV filter.

The material of the organic liquid UV filter is not limited as long as it is organic. If two or more organic liquid UV filters are used, the material(s) of the organic liquid UV filters may be the same as or different from each other.

Amongst the organic liquid UV filter, we can mention:
- Cinnamic derivatives: Ethylhexyl methoxycinnamate, marketed in particular under the trademark "Parsol MCX" by Hoffmann-La Roche; isopropyl methoxycinnamate; isopropoxy methoxycinnamate; isoamyl methoxycinnamate, marketed under the trademark "Neo Heliopan E 1000" by Haarmann and Reimer; cinoxate (2-ethoxyethyl-4-methoxy cinnamate); DEA methoxycinnamate; diisopropyl methylcinnamate; and glyceryl ethylhexanoate dimethoxycinnamate.
- Salicylic derivatives: Homosalate (homomentyl salicylate), marketed under the trademark "Eusolex HMS" by Rona/EM Industries; ethylhexyl salicylate, marketed under the trademark "Neo Heliopan OS" by Haarmann and Reimer; glycol salicylate; butyloctyl salicylate; phenyl salicylate; dipropyleneglycol salicylate, marketed under the trademark "Dipsal" by Scher; and TEA salicylate, marketed under the trademark "Neo Heliopan TS" by Haarmann and Reimer.
- β,β-Diphenylacrylate derivatives: Octocrylene, marketed in particular under the trademark "Uvinul N539" by BASF; and Etocrylene, marketed in particular under the trademark "Uvinul N35" by BASF.
- Polyorganosiloxane comprising benzalmalonate functional groups, such as polysilicone-15, marketed under the trademark "Parsol SLX" by Hoffmann-LaRoche.

The preferred organic liquid UV filter(s) may be selected from:
ethylhexyl methoxycinnamate, homosalate, ethylhexyl salicylate, ethylhexyl triazone, octocrylene, drometrizole trisiloxane, n-hexyl 2-(4-diethylamino-2-hydroxybenzoyl)benzoate, terephthalylidene dicamphor sulfonic acid, and bis-ethylhexyloxyphenol methoxyphenyl triazine.

The UV filter(s) may be present in the composition used for the present invention in a content ranging from 1 to 40% by weight, preferably ranging from 5 to 35% by weight, and more preferably from 10 to 30% by weight, relative to the total weight of the composition.

The composition according to the present invention may comprise water.

The amount of water is not limited, and may be from 10 to 90% by weight, preferably from 20 to 80% by weight, and more preferably from 30 to 70% by weight, relative to the total weight of the composition according to the present invention.

The composition used for the present invention may preferably be used as a cosmetic composition, and more preferably a skin cosmetic composition.

The composition used for the present invention may further comprise at least one additional ingredient, such as a conventional cosmetic ingredient, which may be chosen, for example, from fillers, hydrophilic or lipophilic gelling and/or thickening agents, surfactants, antioxidants, fragrances, preservatives, neutralizing agents, vitamins, moisturizing agents, self-tanning compounds, antiwrinkle active agents, emollients, hydrophilic or lipophilic active agents, agents for combating pollution and/or free radicals, sequestering agents, film-forming agents, dermo-decontracting active agents, soothing agents, agents which stimulate the synthesis of dermal or epidermal macromolecules and/or which prevent their decomposition, antiglycation agents, agents which combat irritation, desquamating agents, depigmenting agents, antipigmenting agents, propigmenting agents, NO-synthase inhibitors, agents which stimulate the proliferation of fibroblasts and/or keratinocytes and/or the differentiation of keratinocytes, agents which act on microcirculation, agents which act on energy metabolism of the cells, healing agents, and mixtures thereof.

The composition used for the present invention may be in various forms, for example, suspensions, dispersions, solutions, gels, emulsions, such as oil-in-water (O/W), water-in-oil (W/O), and multiple (e.g., W/O/W, polyol/O/W, and O/W/O) emulsions, creams, foams, sticks, dispersions of vesicles, for instance, of ionic and/or nonionic lipids, two-phase and multi-phase lotions, sprays, powders, and pastes. The composition may include an aqueous phase or water. On the other hand, the composition may be anhydrous, for example, it can be an anhydrous paste or stick. The composition may also be a leave-on or leave-off composition.

According to one embodiment, the composition used for the present invention may be in the form of a powdery composition or a liquid or solid composition, such as an oily-solid cosmetic composition or an anhydrous composition.

The term "anhydrous composition" means a composition containing less than 1% by weight of water, or even less than 0.5% by weight of water, and especially free of water, the water not being added during the preparation of the composition, but corresponding to the residual water provided by the mixed ingredients.

According to another embodiment, the composition used for the present invention may be in the form of, for example, a compact powder, a lotion or a cosmetic water, a serum, a milk, a cream, a base foundation, an undercoat, a make-up base coat, a foundation, a face powder, cheek rouge, a lipstick, a lip cream, an eye shadow, an eyeliner, a loose powder, a concealer, a nail coat, mascara, a sunscreen, a cleanser, and the like.

It is to be understood that a person skilled in the art can choose the appropriate presentation form, as well as its method of preparation, on the basis of his/her general knowledge, taking into account the nature of the constituents used, for example, their solubility in the vehicle, and the application envisaged for the composition.

### [Anti-Pollution Agent]

The particle and composition according to the present invention may be used as an anti-pollution agent.

Thus, the present invention also relates to a particle or a composition, preferably a cosmetic composition, comprising the particle, for use in protecting keratin materials from pollutants, wherein
the particle has
a wet point for oil being at least 100 ml/100 g, preferably at least 150 ml/100 g, and more preferably at least 200 ml/100 g, and
a wet point for water being at least 100 ml/100 g, preferably at least 200 ml/100 g, and more preferably at least 300 ml/100 g.

It may be preferable that the keratin materials be skin, more preferably face, and that the skin be protected from damage caused by pollutants selected from the group consisting of oily skin, dehydration of skin, alteration of desquamation, squalene decrease, vitamin E decrease, pigmentation, pore problems such as clogged pores, dilated pores, acne and black heads, loss of dry/oily balance, dull skin, aging, and lactic acid increase.

As the particle, the particle explained above can be used. The amount of the particle in the composition used for the present invention may be from 0.01 to 20% by weight, and preferably from 0.1 to 15% by weight, relative to the total weight of the composition.

The pollutants and keratin materials are already explained above.

### [Use]

The present invention also relates to a use of at least one particle or at least one composition, preferably cosmetic composition, comprising the at least one particle, for protecting keratin materials from pollutants, wherein the particle has
a wet point for oil being at least 100 ml/100 g, preferably at least 150 ml/100 g, and more preferably at least 200 ml/100 g, and
a wet point for water being at least 100 ml/100 g, preferably at least 200 ml/100 g, and more preferably at least 300 ml/100 g,
wherein the keratin materials are hair.

As the particle, the particle explained above can be used. The amount of the particle in the composition used in the present invention may be from 0.01 to 20% by weight, and preferably from 0.1 to 15% by weight, relative to the total weight of the composition.

The pollutants and keratin materials are already explained above.

The use according to the present invention can be performed by topically applying the particle(s) or the composition including the particle(s) onto the keratin materials.

The amount of the particle(s) and the composition used for the present invention is not limited. However, for example, from 0.1 g to 10 g of the particle(s) or the composition may be used.

### EXAMPLES

The present invention will be described in more detail by way of examples, which however should not be construed as limiting the scope of the present invention.

### Examples 1-3 and Comparative Examples 1-4

### [Preparation 1]

Each of the powder shown in Table 1 was dispersed in water such that the amount of the powder was 1.0 wt% to prepare aqueous dispersions of Examples 1-3 and Comparative Examples 1-4.

### [Carbon Black Capture Evaluation]

Carbon black was used as a representative of pollutants.

Carbon black was dispersed into water such that the amount of carbon black was 0.5 wt% to prepare an aqueous dispersion of carbon black (Carbon Black Dispersion).

0.2 g of each of the dispersions according to Examples 1-3 and Comparative Examples 1-4 was mixed with 1.0 g of the Carbon Black Dispersion at room temperature. 20 ml of the obtained mixture was dropped onto a glass plate and dried for 30 minutes at room temperature. The color of the dried mixture was compared with that of a control wherein 20 ml of the Carbon Black Dispersion was dropped onto a glass plate and dried for 30 minutes at room temperature. It was evaluated whether the color of the dried mixture was different from the color of the control in accordance with the following criteria.
No: No difference
Yes: The color of the dried mixture was more transparent than that of the control

The results are shown in Table 1.

**Table 1**

| | Powder | Shape | Mean Size (µm) | WP Oil (ml/100 g) | WP Water (ml/100 g) | Color Change |
|---|---|---|---|---|---|---|
| Ex. 1 | Cellulose beads (Cellulobeads USF, Daito Kasei) | Sphere | 4.0 | 296.0 | 400.8 | Yes |
| Ex. 2 | Silica(Sunspheres H33, AGC SI-TECH) | Sphere | 2.9 | 370.0 | 310.6 | Yes |
| Ex. 3 | Boron nitride (SHP3, Mizushima Ferroalloy) | Potato | 6.7 | 104.3 | 186.4 | Yes |
| Comp. Ex. 1 | Barium sulfate (LLD-5, Daito Kasei) | Potato | 4.3 | 23.9 | 30.7 | No |
| Comp. Ex. 2 | Nylon-12 (SP-500, Toray) | Sphere | 4.6 | 68.0 | 74.0 | No |
| Comp. Ex. 3 | PTFE (Ceridust 9205F, Clariant) | Sphere | 3.5 | 45.0 | 0 | No |
| Comp. Ex. 4 | Silicone resin (Tospearl 145A, Momentive performance materials) | Sphere | 4.6 | 50.0 | 0 | No |

| | | | | | | |
|---|---|---|---|---|---|---|
| Mean Size: Number-Average Primary Particle Size WP Oil: Wet Point for Oil WP Water: Wet Point for Water | | | | | | |

### (Wet Point for Oil)

The wet point for oil was determined by the following protocol.
(1) 2g of the powder component was kneaded with a spatula on a glass plate while adding isononyl isononanoate with a viscosity of 9 cP at 25°C and a density of 0.853 g/ml.
(2) When the powder component became completely wet and started to form a paste, the weight of the added oil was determined as the weight of wet point.
(3) The wet point for oil was calculated from the equation: Wet point for oil (ml/100 g) = {(the weight of wet point)/2 g}X100/the density of oil.

### (Wet Point for Water)

The wet point for water was determined by the following protocol.
(1) 2 g of the powder component was kneaded with a spatula on a glass plate while adding water with a density of 0.998 g/ml.
(2) When the powder component became completely wet and started to form a paste, the weight of the added water was determined as the weight of wet point.
(3) The wet point for water was calculated from the equation: Wet point for water (ml/100 g) = {(the weight of wet point)/2 g}X100/the density of water.

### (Results)

The color change in Table 1 demonstrates that Examples 1-3 in which a powder with both a wet point for oil of 100 mg/100 g or more and a wet point for water of 100 mg/100 ml or more captured carbon black, while Comparative Examples 1-4 in which a powder with a wet point for oil of less than 100 mg/100 g or a wet point for water of less than 100 mg/100 ml could not capture carbon black.

Accordingly, it is clear that a powder with both a wet point for oil of 100 mg/100 g or more and a wet point for water of 100 mg/100 ml or more can protect keratin material such as skin from damage caused by pollutants.

### Example 4 and Comparative Example 5

### [Preparation 2]

5.0 g of Cellulose beads used in Example 1 was mixed with 90 g of Solution B (10% polyglyceryl-10 laurate:decaglyceryl monolaurate aqueous solution) to prepare an aqueous dispersion according to Example 4.

### [Benzopyrene Capture Evaluation]

Benzopyrene is one of PAHs, and was used as another representative of pollutants.

The above aqueous dispersion according to Example 4 was further mixed with 5.0 g of Solution A (1% benzopyrene acetone solution). 5g of the obtained mixture was dried. The residue obtained by drying was re-dispersed in 5g of water, and filtered with a 2 µm membrane filter in order to remove the cellulose beads. The quantity of the benzopyrene in the filtrate was measured by gas chromatography.

As Comparative Example 5, 5.0 g of Solution A and 95 g of Solution B were mixed. 5g of the obtained mixture was dried. The residue obtained by drying was re-dissolved in 5g of water, and filtered with a 2 µm membrane filter. The quantity of the benzopyrene in the filtrate was measured by gas chromatography.

The change in the content of benzopyrene was evaluated. The results are shown in Table 2. The numerical values for the amounts of the ingredients shown in Table 2 are all based on "% by weight" as active raw materials.

**Table 2**

| | | Example 4 | Comparative Example 5 |
|---|---|---|---|
| Solution A | Benzopyrene | 0.05 | 0.05 |
| | Acetone | 4.95 | 4.95 |
| Solution B | Water | 81 | 85.5 |
| | Polyglyceryl-10 Laurate: Decaglyceryl Monolaurate | 9 | 9.5 |
| Porous Particle | Cellulose beads | 5.00 | - |
| Benzopyrene Content Reduction | | -30% | |

### (Results)

The benzopyrene content reduction in Table 2 demonstrates that a cellulose powder with both a wet point for oil of 100 mg/100 g or more and a wet point for water of 100 mg/100 ml or more captured benzopyrene.

Accordingly, it is clear that a powder with both a wet point for oil of 100 mg/100 g or more and a wet point for water of 100 mg/100 ml or more can protect keratin material such as skin from damage caused by pollutants.

### Example 5 and Comparative Example 6

### [Preparation 3]

5.0 g of Cellulose beads used in Example 1 was mixed with 90 g of Solution B (10% polyglyceryl-10 laurate:decaglyceryl monolaurate aqueous solution) to prepare an aqueous dispersion according to Example 5.

### [Oleic Acid Oxidation Inhibition Evaluation]

Benzopyrene was used as a representative of pollutants. Oleic acid was used as a representative of sebum.

The above aqueous dispersion according to Example 5 was further mixed with 5.0 g of Solution C (0.2% benzopyrene and 2% oleic acid acetone solution).

1g of the obtained mixture was dried at 50 °C for one night. 5 g of water was added to the dried mixture, and the dried mixture was re-dispersed by hand shaking. The obtained dispersion was irradiated with UV ray with Suntest CPS (TOYO SEIKI, 765W/m²) for 15 minutes. The level of hydroperoxide was analyzed with an LPO kit (Lipid Hydroperoxide Assay kit from Cayman).

As Comparative Example 6, 5.0 g of Solution C and 95 g of Solution B were mixed. 1g of the obtained mixture was dried at 50 °C for one night. 5 g of water was added to the dried mixture, and the dried mixture was re-dispersed by hand shaking. The obtained dispersion was irradiated with UV ray with Suntest CPS (TOYO SEIKI, 765W/m²) for 15minutes. The level of hydroperoxide was analyzed with an LPO kit (Lipid Hydroperoxide Assay kit from Cayman).

| | | Example 5 | Comparative Example 6 |
|---|---|---|---|
| Solution C | Benzopyrene | 0.01 | 0.01 |
| | Oleic acid | 0.1 | 0.1 |
| | Acetone | 4.89 | 4.89 |
| Solution B | Water | 81 | 76 |
| | Polyglyceryl-10 Laurate: Decaglyceryl Monolaurate | 9 | 9 |
| Porous Particle | Cellulose beads | 5.00 | - |

The results are shown in Table 3

**Table 3**

| | | Hydroperoxide Concentration (µM) |
|---|---|---|
| Example 5 | After UV irradiation | 10.4 |
| Comparative Example 6 | After UV irradiation | 14.2 |

### (Results)

The increase of hydroperoxide level after UV irradiation of Example 5 (benzopyrene solution with cellulose beads) was lower than that of Comparative Example 6 (benzopyrene solution without cellulose beads). This is based on the capture of benzopyrene by cellulose beads because benzopyrene is known as a substance which promotes oxidation of sebum, and the capture of benzopyrene could reduce the possibility of contact between benzopyrene and oleic acid.

Accordingly, it is clear that a powder with both a wet point for oil of 100 mg/100 g or more and a wet point for water of 100 mg/100 ml or more can protect keratin material such as skin from damage, in particular damage due to the production of reactive oxygen species, caused by pollutants.

### Examples 6-10 and Comparative Examples 7-10

### [Preparation 4]

The following cosmetic compositions according to Examples 6-10 and Comparative Examples 7-10, shown in Table 4, were prepared by mixing the ingredients shown in Table 4. The numerical values for the amounts of the ingredients shown in Table 4 are all based on "% by weight" as active raw materials. The cellulose beads in Table 4 were the same as those used in Example 1.

### [Carbon Black Capture Evaluation]

Carbon black was used as a representative of pollutants.

Carbon black was dispersed into water such that the amount of carbon black was 0.5 wt% to prepare an aqueous dispersion of carbon black (Carbon Black Dispersion). Also, each of compositions according to Examples 6-10 and Comparative Examples 7-10 was dispersed into water such that the amount of each composition was 10 wt% to prepare aqueous dispersions of Examples 6-10 and Comparative Examples 7-10.

0.2g of each of the dispersions of Examples 6-10 and Comparative Examples 7-10 was mixed with 1.0 g of the Carbon Black Dispersion at room temperature. 20 µl of the obtained mixture was dropped onto a glass plate and dried for 30 minutes at room temperature. The color of the dried mixture was compared with that of a control wherein 20 µl of the Carbon Black Dispersion was dropped onto a glass plate and dried for 30 minutes at room temperature. It was evaluated whether the color of the dried mixture was different from the color of the control in accordance with the following criteria.
No: No difference
Yes: The color of the dried mixture was more transparent than that of the control

The results are shown in Table 4

**Table 4**

| | Comp. Ex. 7 | Ex. 6 | Ex. 7 | Comp. Ex. 8 | Ex. 8 | Comp. Ex. 9 | Ex. 9 | Comp. Ex. 10 | Ex. 10 |
|---|---|---|---|---|---|---|---|---|---|
| | W/O Emulsion (Leave on) | | | O/W Emulsion (Leave on) | | Aqueous Solution | | O/W Emulsion (Rinse off) | |
| KOH | - | - | - | - | - | 0.68 | 0.68 | 0.85 | 0.85 |
| Tetrasodium EDTA | - | - | - | - | - | 0.1 | 0.1 | - | - |
| Disodium EDTA | 0.1 | 0.1 | 0.1 | 0.05 | 0.05 | - | - | - | - |
| Sodium Citrate | - | - | - | - | - | 0.15 | 0.15 | - | - |
| Sodium Chloride | 0.1 | 0.1 | 0.1 | - | - | - | - | - | - |
| Triethanolamine | 1.35 | 1.35 | 1.35 | 2.48 | 2.48 | - | - | - | - |
| Arginine(and)Mannitol(and)Pyridoxine | - | - | - | 0.1 | 0.1 | - | - | - | - |
| HCl(and)Histidine HCl(and)RNA(and)Disodium | | | | | | | | | |
| Adenosine Triphosphate(and)Phenylalanine(and)Tvrosine | | | | | | | | | |
| Tromethamine | - | - | - | 0.245 | 0.245 | - | - | - | - |
| Citric Acid | - | - | - | - | - | 0.075 | 0.075 | - | - |
| Cetyl Alcohol | - | - | - | 0.7 | 0.7 | - | - | - | - |
| Diisopropyl Sebacate | 4.5 | 4.5 | 4.5 | - | - | - | - | - | - |
| Dicaprylyl Carbonate | 5 | 5 | 5 | - | - | - | - | - | - |
| Polyglyceryl-6 Polvricinoleate | 0.5 | 0.5 | 0.5 | - | - | - | - | - | - |
| **Cellulose beads** | - | 0.5 | 5 | - | 5 | - | 5 | - | 5 |
| Titanium dioxide(and)aluminum hydroxide(and)stearic acid | - | - | - | 4 | 4 | - | - | - | - |
| PEG-32 | 0.5 | 0.5 | 0.5 | - | - | 0.55 | 0.55 | - | - |
| Xanthan Gum | - | - | - | 0.1 | 0.1 | 0.05 | 0.05 | - | - |
| Nylon-12 | - | - | - | 1 | 1 | - | - | - | - |
| Carbomer | - | - | - | 0.3 | 0.3 | - | - | - | - |
| Acrylamide/Sodium Acryloyldimethyltaurate Copolymer(and)Isohexadecane(and)Polysorbate 80 | - | - | - | 0.5 | 0.5 | - | - | - | - |
| Polyquaternium-39 | - | - | - | - | - | - | - | 5 | 5 |
| Acrylates/Vinyl Neodecanoate Crosspolymer | - | - | - | - | - | - | - | 9 | 9 |
| (continued) | | | | | | | | | |
| (continued) | | | | | | | | | |
| Acrylonitrile/Methyl Methacrylate/Vinylidene Chloride Copolymer | 0.15 | 0.15 | 0.15 | - | - | - | - | - | - |
| Phenoxyethanol | 0.7 | 0.7 | 0.7 | 0.5 | 0.5 | 0.3 | 0.3 | 0.5 | 0.5 |
| Dimethicone | 10 | 10 | 10 | 0.5 | 0.5 | - | - | - | - |
| Cyclopentasiloxane | - | - | - | 4 | 4 | - | - | - | - |
| Polymethylsilsesquioxane | 2 | 2 | 2 | - | - | - | - | - | - |
| PEG-8 | - | - | - | - | - | 1.65 | 1.65 | - | - |
| Dipropylene Glycol | - | - | - | - | - | 3.3 | 3.3 | - | - |
| Butylene Glycol | - | - | - | - | - | 9 | 9 | - | - |
| Alcohol Denat. | - | - | - | 3 | 3 | 3.3 | 3.3 | - | - |
| Water | qs100 | qs100 | qs100 | qs100 | qs100 | qs100 | qs100 | qs100 | qs100 |
| Glycerin | - | - | - | 4 | 4 | 4 | 4 | 10 | 10 |
| Propylene Glycol | 5 | 5 | 5 | 3.8 | 3.8 | - | - | - | - |
| Ethylhexyl Methoxycinnamate | 6.75 | 6.75 | 6.75 | 7.5 | 7.5 | - | - | - | - |
| Ethylhexyl Triazone | 1 | 1 | 1 | - | - | - | - | - | - |
| Terephthalylidene Dicamphor Sulfonic Acid | 7.5 | 7.5 | 7.5 | 12 | 12 | - | - | - | - |
| Homosalate | 5 | 5 | 5 | - | - | - | - | - | - |
| Drometrizole Trisiloxane | 3 | 3 | 3 | 3 | 3 | - | - | - | - |
| Bis-Ethvlhexvloxvphenol Methoxyphenyl Triazine | 3 | 3 | 3 | - | - | - | - | - | - |
| Diethylamino Hydroxybenzoyl Hexyl Benzoate | 3 | 3 | 3 | - | - | - | - | - | - |
| Stearic Acid | - | - | - | 2 | 2 | - | - | - | - |
| Glyceryl Stearate(and)PEG-100 Stearate | - | - | - | 1.5 | 1.5 | - | - | - | - |
| Potassium Cetyl Phosphate | - | - | - | 1 | 1 | - | - | - | - |
| Lauryl Betaine(and)Sodium Chloride | - | - | - | - | - | - | - | 7.33 | 7.33 |
| Glycereth-25 PCA Isostearate | - | - | - | - | - | 0.3 | 0.3 | - | - |
| Sodium Cocoyl Sarcosinate | - | - | - | 1 | 1 | - | - | - | - |
| Potassium Cocoyl Glycinate(and) Potassium Cocoate | - | - | - | - | - | - | - | 20 | 20 |
| Color Change | No | Yes | Yes | No | Yes | No | Yes | No | Yes |

### (Results)

The color change in Table 4 demonstrates that a cellulose powder with both a wet point for oil of 100 mg/100 g or more and a wet point for water of 100 mg/100 ml or more in a variety of cosmetic formulations captured carbon black.

Accordingly, it is clear that a powder with both a wet point for oil of 100 mg/100 g or more and a wet point for water of 100 mg/100 ml or more can protect keratin material such as skin from damage caused by pollutants.

### Examples 11 and 12 and Comparative Example 11

### [Preparation 5]

The following cosmetic compositions according to Examples 11 and 12 and Comparative Example 11, shown in Table 5, were prepared by mixing the ingredients shown in Table 5. The numerical values for the amounts of the ingredients shown in Table 5 are all based on "% by weight" as active raw materials. The cellulose beads in Table 5 were the same as those used in Example 1.

**Table 5**

| | Comp. Ex. 11 | Ex. 11 | Ex. 12 |
|---|---|---|---|
| Disodium EDTA | 0.1 | 0.1 | 0.1 |
| Sodium Chloride | 0.1 | 0.1 | 0.1 |
| Triethanolamine | 1.35 | 1.35 | 1.35 |
| Diisopropyl Sebacate | 4.5 | 4.5 | 4.5 |
| Dicaprylyl Carbonate | 5 | 5 | 5 |
| Polyglyceryl-6 Polyricinoleate | 0.5 | 0.5 | 0.5 |
| **Cellulose beads** | | 0.55 | 5 |
| Fragrance | 0.3 | 0.3 | 0.3 |
| Acrylonitrile/Methyl Methacrylate/Vinylidene Chloride Copolymer | 0.15 | 0.15 | 0.15 |
| Phenoxyethanol | 0.7 | 0.7 | 0.7 |
| Dimethicone | 10 | 10 | 10 |
| Polymethylsilsesquioxane | 2 | 2 | 2 |
| Water | qsp100 | qsp100 | qsp100 |
| Ethylhexyl Methoxycinnamate | 6.75 | 6.75 | 6.75 |
| Terephthalylidene Dicamphor Sulfonic Acid | 7.5 | 7.5 | 7.5 |
| Homosalate | 5 | 5 | 5 |
| Drometrizole Trisiloxane | 3 | 3 | 3 |
| Diethylamino Hydroxybenzoyl Hexyl Benzoate | 3 | 3 | 3 |

### [Cosmetic Effect Evaluation]

0.15 ml of each of the dispersions of Examples 11 and 12 and was applied onto half of the face of 6 Japanese women, and 0.15 ml of Comparative Example 11 was applied onto the other half of the face of the 6 Japanese women. The cosmetic effects regarding "pore problems", "loss of balance", "dull skin" and "ageing skin" provided by the dispersions of Examples 11 and 12 were compared with those provided by the dispersion of Comparative Example 11 in accordance with the following criteria.
- +++: Very different/ Very easy to see the difference with a quick look
- ++: Different / Easy to see the difference
- +: A little different / Not very easy to see the difference, need to look carefully
- 0: No difference

The "pore problems", "loss of balance", "dull skin" and "ageing skin" relate to pollutions, and specifically relate to the following.
Pore Problems: clogged pores, dilated pores, acne, or black heads
Loss of Balance: unbalanced dry/oily areas
Dull Skin: uneven skin tone or relief
Ageing Skin: premature ageing signs such as wrinkles

The results are shown in Tables 6 and 7.

**Table 6**

| Skin concern relating to pollution | | Comp. Ex. 11 | Ex. 12 |
|---|---|---|---|
| | Cellulobeads dosage | 0% | 5.00% |
| Pore Problems | Hide dilated pores | | ++ |
| | Marking dilated pores | ++ | |
| Loss of Balance | Greasy/oily feeling | + | |
| | Greasv/oilv sensation | + | |
| | Sticky sensation | + | |
| Dull Skin | Skin color looks evener | | + |
| | Marking skin defect (relief) | ++ | |
| Ageing Skin | Hide dehydration lines under eye and cheek | | ++ |
| | Marking dehydration line under eye and cheek | + | |

**Table 7**

| Skin concern relating to pollution | | Comp. Ex. 11 | Ex. 11 |
|---|---|---|---|
| | Cellulobeads dosage | 0% | 0.55% |
| Pore Problems | Hide dilated pores | | + |
| | Marking dilated pores | + | |
| Loss of Balance | Greasv/oilv feeling | + | |
| | Greasy/oily sensation | + | |
| | Sticky sensation | 0 | |
| Dull Skin | Skin color looks evener | 0 | |
| | Marking skin defect (relief) | + | |
| Ageing Skin | Hide dehydration line under eye and cheek | 0 | |
| | Marking dehydration line under eye and cheek | 0 | |

### (Results)

The test results shown in Tables 6 and 7 show that a cellulose powder with both a wet point for oil of 100 mg/100 g or more and a wet point for water of 100 mg/100 ml or more can provide skin with cosmetic effects based on the protection of the skin from pollutants.

Specifically, the comparison between Example 12 and Comparative Example 11 shows that the cellulose powder in an amount of 5.0% by weight can provide skin benefits against pore problems, loss of balance, dull skin, and aging skin, which relate to pollution. Similarly, the comparison between Example 11 and Comparative Example 11 shows that the cellulose powder in an amount of 0.55% by weight can provide skin benefits against pore problems, loss of balance, and dull skin, which relate to pollution.

## Claims

1. A non-therapeutic method, preferably a cosmetic method, of protecting keratin materials from pollutants, comprising:
applying the keratin materials with at least one composition, preferably at least one cosmetic composition, comprising at least one particle having
a wet point for oil being at least 100 ml/100 g, preferably at least 150 ml/100 g, and more preferably at least 200 ml/100 g, and
a wet point for water being at least 100 ml/100 g, preferably at least 200 ml/100 g, and more preferably at least 300 ml/100 g,
wherein the keratin materials are hair.

2. The method according to Claim 1, wherein the pollutants are aerial pollutants.

3. The method according to Claim 1 or 2, wherein the pollutants are selected from the group consisting of carbon black, carbon oxides, nitrogen oxides, sulfur oxides, hydrocarbons, organic volatiles, heavy metals, PM2.5, PM10 and mixtures thereof.

4. The method according to any one of Claims 1 to 3, wherein the number-average primary particle size of the particle is 50 µm or less, preferably 30 µm or less, and more preferably 10 µm or less.

5. The method according to any one of Claims 1 to 4, wherein the ratio of the wet point for water/the wet point for oil is 5 or less, preferably 4 or less, and more preferably 2 or less.

6. The method according to any one of Claims 1 to 5, wherein the particle is porous.

7. The method according to any one of Claims 1 to 6, wherein the particle is selected from the group consisting of polysaccharides, boron compounds, metal compounds, polymers, perlites, and mixtures thereof.

8. The method according to any one of Claims 1 to 6, wherein the particle comprises at least one polysaccharide, preferably cellulose.

9. The method according to any one of Claims 1 to 6, wherein the particle comprises boron nitride.

10. The method according to any one of Claims 1 to 9, wherein the amount of the particle in the composition is from 0.01 to 20% by weight, preferably from 0.1 to 15% by weight, and more preferably from 1.0 to 10% by weight relative to the total weight of the composition.

11. A particle or a composition, preferably a cosmetic composition, comprising the
particle, for use in protecting keratin materials from pollutants, wherein
the particle has
a wet point for oil being at least 100 ml/100 g, preferably at least 150 ml/100 g, and more preferably at least 200 ml/100 g, and
a wet point for water being at least 100 ml/100 g, preferably at least 200 ml/100 g, and more preferably at least 300 ml/100 g.

12. The particle or composition according to Claim 11, wherein the keratin materials are selected from the group consisting of skin, scalp, lips and hair.

13. The particle or composition according to Claim 11 or 12, wherein
the keratin materials are skin, preferably face, and
the skin is protected from damage caused by pollutants selected from the group consisting of oily skin, dehydration of skin, alteration of desquamation, squalene decrease, vitamin E decrease, pigmentation, pore problems such as clogged pores, dilated pores, acne and black heads, loss of dry/oily balance, dull skin, aging, and lactic acid increase.

14. A use of at least one particle or at least one composition, preferably cosmetic composition, comprising the at least one particle, for protecting keratin materials from pollutants, wherein the particle has
a wet point for oil being at least 100 ml/100 g, preferably at least 150 ml/100 g, and more preferably at least 200 ml/100 g, and
a wet point for water being at least 100 ml/100 g, preferably at least 200 ml/100 g, and more preferably at least 300 ml/100 g,
wherein the keratin materials are hair.

## Patentansprüche

1. Nichttherapeutisches Verfahren, vorzugsweise kosmetisches Verfahren, zum Schützen von Keratinmaterialien vor Schadstoffen, Folgendes umfassend:
Anwenden der Keratinmaterialien mit mindestens einer Zusammensetzung, vorzugsweise mindestens einer kosmetischen Zusammensetzung, enthaltend mindestens einen Partikel mit einem Nasspunkt für Öl bei mindestens 100 ml/100 g, vorzugsweise bei mindestens 150 m1/ 100 g und besonders bevorzugt bei mindestens 200 m1/ 100 g und mit einem Nasspunkt für Wasser bei mindestens 100 ml/100 g, vorzugsweise bei mindestens 200 ml/100 g und besonders bevorzugt bei mindestens 300 ml/100 g, wobei die Keratinmaterialien Haare sind.

2. Verfahren nach Anspruch 1, wobei die Schadstoffe Luftschadstoffe sind.

3. Verfahren nach Anspruch 1 oder 2, wobei die Schadstoffe aus der Gruppe ausgewählt sind, bestehend aus Ruß, Kohlenoxiden, Stickoxiden, Schwefeloxiden, Kohlenwasserstoffen, flüchtigen organischen Stoffen, Schwermetallen, Feinstaub PM2,5, Feinstaub PM10 und Mischungen davon.

4. Verfahren nach einem der Ansprüche 1 bis 3, wobei der Zahlenmittelwert der Primärpartikelgröße des Partikels 50 µm oder weniger, vorzugsweise 30 µm oder weniger und besonders bevorzugt 10 µm oder weniger ist.

5. Verfahren nach einem der Ansprüche 1 bis 4, wobei das Verhältnis Nasspunkt für Wasser/Nasspunkt für Öl 5 oder weniger, vorzugsweise 4 oder weniger und besonders bevorzugt 2 oder weniger ist.

6. Verfahren nach einem der Ansprüche 1 bis 5, wobei der Partikel porös ist.

7. Verfahren nach einem der Ansprüche 1 bis 6, wobei der Partikel aus der Gruppe ausgewählt ist, bestehend aus Polysacchariden, Borverbindungen, Metallverbindungen, Polymeren, Perliten und Mischungen davon.

8. Verfahren nach einem der Ansprüche 1 bis 6, wobei der Partikel mindestens ein Polysaccharid, vorzugsweise Cellulose, enthält.

9. Verfahren nach einem der Ansprüche 1 bis 6, wobei der Partikel Bornitrid enthält.

10. Verfahren nach einem der Ansprüche 1 bis 9, wobei die Menge des Partikels in der Zusammensetzung zwischen 0,01 und 20 Masse-%, vorzugsweise zwischen 0,1 und 15 Masse-% und besonders bevorzugt zwischen 1,0 bis 10 Masse-% in Bezug auf die Gesamtmasse der Zusammensetzung ist.

11. Partikel oder Zusammensetzung, vorzugsweise kosmetische Zusammensetzung, die den Partikel enthält, zur Verwendung beim Schutz von Keratinmaterialien vor Schadstoffen, wobei der Partikel einen Nasspunkt für Öl bei mindestens 100 ml/100 g, vorzugsweise bei mindestens 150 ml/100 g und besonders bevorzugt bei mindestens 200 ml/100 g und einen Nasspunkt für Wasser bei mindestens 100 ml/100 g, vorzugsweise bei mindestens 200 ml/100 g und besonders bevorzugt bei mindestens 300 ml/100 g aufweist.

12. Partikel oder Zusammensetzung nach Anspruch 11, wobei die Keratinmaterialien aus der Gruppe ausgewählt sind, bestehend aus Haut, Kopfhaut, Lippen und Haar.

13. Partikel oder Zusammensetzung nach Anspruch 11 oder 12, wobei die Keratinmaterialien Haut, vorzugsweise Gesichtshaut, sind und die Haut vor Schäden geschützt wird, die durch Schadstoffe bewirkt werden, die aus der Gruppe ausgewählt sind, bestehend aus fettiger Haut, trockener Haut, Veränderung der Desquamation, Squalen-Absenkung, Vitamin-E-Absenkung, Pigmentierung, Porenproblemen, wie beispielsweise verstopfte Poren, erweiterte Poren, Akne und Mitesser, Verlust des Trocken- /Fettgleichgewichts, matter Haut, Alterung und Milchsäure-Erhöhung.

14. Verwendung von mindestens einem Partikel oder mindestens einer Zusammensetzung, vorzugsweise einer kosmetischen Zusammensetzung, die den mindestens einen Partikel enthält, zum Schützen von Keratinmaterialien vor Schadstoffen, wobei der Partikel einen Nasspunkt für Öl bei mindestens 100 ml/100 g, vorzugsweise bei mindestens 150 ml/100 g und besonders bevorzugt bei mindestens 200 ml/100 g und einen Nasspunkt für Wasser bei mindestens 100 ml/100 g, vorzugsweise bei mindestens 200 ml/100 g und besonders bevorzugt bei mindestens 300 ml/100 g aufweist, wobei die Keratinmaterialien Haar sind.

## Revendications

1. Procédé non thérapeutique, préférablement procédé cosmétique, de protection de matières kératiniques contre des polluants, comprenant :
l'application d'au moins une composition aux matières kératiniques, préférablement d'au moins une composition cosmétique, comprenant au moins une particule possédant
un point humide pour l'huile qui est d'au moins 100 ml/100 g, préférablement d'au moins 150 ml/100 g, et plus préférablement d'au moins 200 ml/100 g, et
un point humide pour l'eau qui est d'au moins 100 ml/100 g, préférablement d'au moins 200 ml/100 g, et plus préférablement d'au moins 300 ml/100 g,
les matières kératiniques étant des cheveux.

2. Procédé selon la revendication 1, les polluants étant des polluants aériens.

3. Procédé selon la revendication 1 ou 2, les polluants étant choisis dans le groupe constitué par le noir de carbone, des oxydes de carbone, des oxydes d'azote, des oxydes de soufre, des hydrocarbures, des composés organiques volatils, des métaux lourds, PM2.5, PM10 et des mélanges correspondants.

4. Procédé selon l'une quelconque des revendications 1 à 3, la taille moyenne en nombre de particule primaire de la particule étant de 50 µm ou moins, préférablement de 30 µm ou moins, et plus préférablement de 10 µm ou moins.

5. Procédé selon l'une quelconque des revendications 1 à 4, le rapport du point humide pour l'eau/le point humide pour l'huile étant de 5 ou moins, préférablement de 4 ou moins, et plus préférablement de 2 ou moins.

6. Procédé selon l'une quelconque des revendications 1 à 5, la particule étant poreuse.

7. Procédé selon l'une quelconque des revendications 1 à 6, la particule étant choisie dans le groupe constitué par des polysaccharides, des composés du bore, des composés métalliques, des polymères, des perlites et des mélanges correspondants.

8. Procédé selon l'une quelconque des revendications 1 à 6, la particule comprenant au moins un polysaccharide, préférablement une cellulose.

9. Procédé selon l'une quelconque des revendications 1 à 6, la particule comprenant du nitrure de bore.

10. Procédé selon l'une quelconque des revendications 1 à 9, la quantité de la particule dans la composition étant de 0,01 à 20 % en poids, préférablement de 0,1 à 15 % en poids, et plus préférablement de 1,0 à 10 % en poids par rapport au poids total de la composition.

11. Particule ou composition, préférablement composition cosmétique, comprenant la particule, pour une utilisation dans la protection de matières kératiniques contre des polluants, la particule possédant
un point humide pour l'huile qui est d'au moins 100 ml/100 g, préférablement d'au moins 150 ml/100 g, et plus préférablement d'au moins 200 ml/100 g, et un point humide pour l'eau qui est d'au moins 100 ml/100 g, préférablement d'au moins 200 ml/100 g, et plus préférablement d'au moins 300 ml/100 g.

12. Particule ou composition selon la revendication 11, les matières kératiniques étant choisies dans le groupe constitué par la peau, le cuir chevelu, les lèvres et les cheveux.

13. Particule ou composition selon la revendication 11 ou 12,
les matières kératiniques étant la peau, préférablement le visage, et
la peau étant protégée contre des dommages causés par des polluants choisis dans le groupe constitué par la peau grasse, la déshydratation de la peau, l'altération de la désquamation, une diminution du squalène, une diminution de la vitamine E, une pigmentation, des problèmes de pores tels que des pores bouchés, des pores dilatés, l'acné et des points noirs, une perte d'équilibre sec/huileux, la peau terne, le vieillissement et une augmentation d'acide lactique.

14. Utilisation d'au moins une particule ou d'au moins une composition, préférablement d'une composition cosmétique, comprenant l'au moins une particule, pour la protection de matières kératiniques contre des polluants, la particule possédant
un point humide pour l'huile qui est d'au moins 100 ml/100 g, préférablement d'au moins 150 ml/100 g, et plus préférablement d'au moins 200 ml/100 g, et un point humide pour l'eau qui est d'au moins 100 ml/100 g, préférablement d'au moins 200 ml/100 g, et plus préférablement d'au moins 300 ml/100 g, les matières kératiniques étant des cheveux.
